Europäisches Patentamt

European Patent Office

Office européen des brevets

(11) Veröffentlichungsnummer: **0 209 733**
A2

# (12) EUROPÄISCHE PATENTANMELDUNG

(21) Anmeldenummer: 86108396.2

(22) Anmeldetag: 19.06.86

(51) Int. Cl.4: **C07C 55/14** , C07C 55/20 , C07C 55/18 , C07C 55/21 , C07C 93/02

(30) Priorität: 21.06.85 DE 3522216

(43) Veröffentlichungstag der Anmeldung:
28.01.87 Patentblatt 87/05

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Pilz, Georg
Ahornweg 4
D-6730 Neustadt(DE)
Erfinder: Buerger, Gert, Dr.
Hoeferstrasse 16
D-6800 Mannheim 23(DE)
Erfinder: Barl, Manfred, Dr.
Pappelstrasse 2
D-6701 Otterstadt(DE)
Erfinder: Thiel, Gerhard
Untergasse 35
D-6700 Ludwigshafen(DE)

(54) Verwendung von Diaminen zur Herstellung von Salzen mit Dicarbonsäuren.

(57) Verwendung von Diaminen der Formel

$H_2N-(R^1-O)_n-R^2-NH_2$ I

in der $R^1$ und $R^2$ jeweils gleich oder verschieden sein können und jeweils für einen Alkylenrest mit 2 bis 4 Kohlenstoffatomen stehen und n eine ganze Zahl von 1 bis 3 bedeutet, zur Herstellung von Salzen mit Dicarbonsäuren, dadurch gekennzeichnet, daß die Diamine der Formel I einen Gehalt an Hydrazin in Form von Hydrazinhydrat haben.

Xerox Copy Centre

## Verwendung von Diaminen zur Herstellung von Salzen mit Dicarbonsäuren

Salze aus Dicarbonsäuren und Diaminen wie Hexamethylendiammoniumadipat werden in großem Umfang hergestellt und dienen als Ausgangsstoffe zur Herstellung von Polyamiden durch Polykondensation. An die Qualität solcher Polyamide werden sehr hohe Ansprüche hinsichtlich der Eigenschaften gestellt. Demzufolge gilt dies auch für die als Ausgangsstoffe verwendeten Salze aus Dicarbonsäuren und Diaminen und die zur Herstellung solcher Salze verwendeten Dicarbonsäuren und Diamine. Es wurden auch schon Salze aus 4,7-Dioxadecan-1,10-diamin, das durch Destillation gereinigt wurde, und Dicarbonsäuren hergestellt. So erhaltene Salze entsprechen jedoch nicht den gestellten Anforderungen. Es ist zwar bekannt, daß man Amine durch Zusatz von geringen Mengen Hydrazin gegen Luftsauerstoff stabilisiert. Ferner ist es aus der US-PS 4 165 335 bekannt, daß man Alkandiaminen Hydrazinhydrat zusetzt, um die Farbqualität der daraus hergestellten Salze zu verbessern. Im Hinblick auf die chemisch andersartige Natur von 4,7-Dioxadecan-1,10-diamin wurde kein Hinweis gegeben, wie zu verfahren ist, um sämtliche erforderlichen Kennzahlen zu erreichen. Dies umso mehr als die nachteiligen Eigenschaften durch geringste Mengen an Verunreinigungen hervorgerufen werden, deren Natur bislang nicht bekannt ist und die mit den herkömmlichen Reinigungsmethoden unter vertretbarem Aufwand bislang nicht entfernt werden konnten.

Es war deshalb die technische Aufgabe gestellt, Salze aus Dicarbonsäuren und Diaminen die Sauerstoffatome in der Kette enthalten, zur Verfügung zu stellen, die hinsichtlich ihrer Farbzahl, Vergilbungszahl, Perjodatzahl, insbesondere der UV-Zahl nach einer Behandlung bei erhöhter Temperatur und weiteren Kennzahlen den gestellten Anforderungen besser entsprechen.

Diese Aufgabe wird gelöst durch Verwendung von Diaminen der Formel I

$H_2N-(R^1-O)_n-R^2-NH_2$ I

in der $R^1$ und $R^2$ gleich oder verschieden sein können und jeweils einen Alkylenrest mit 2 bis 4 Kohlenstoffatomen bezeichnen und n für eine ganze Zahl von 1 bis 3 steht, zur Herstellung von Salzen mit Dicarbonsäuren, dadurch gekennzeichnet, daß die Diamine der Formel I einen Gehalt an —Hydrazin in Form von Hydrazinhydrat haben.

Die Verwendung von Diaminen der Formel I mit einem Gehalt an Hydrazin in Form von Hydrat zur Herstellung von Salzen mit Dicarbonsäuren hat den Vorteil, daß auf einfache Weise die Wirkung von unbekannten Verunreini gungen ausgeschaltet wird und die Kennzahl solcher Salze wie Farbzahl, Vergilbungszahl, UV-Zahl nach der Behandlung bei erhöhter Temperatur sowie Perjodatzahl und weitere Kennzahlen verbessert wird.

Als Diamine verwendet man solche der Formel I

$H_2N-(R^1-O)_n-R^2-NH_2$ I

In der Formel I bedeuten $R^1$ und $R^2$ jeweils gleiche oder verschiedene Alkylenreste mit 2 bis 4 Kohlenstoffatomen. Geeignete Reste sind beispielsweise Ethylenreste, Propylen-1,2-Reste, Propylen-1,3-Reste oder Butylen-1,4-Reste. Bevorzugt sind Ethylen-und Propylenreste. In den Diaminen der Formel I bezeichnet n eine ganze Zahl von 1 bis 3, insbesondere 1 oder 2. Geeignete Diamine sind beispielsweise solche der Formeln II bis VI

$H_2N-(CH_2)_3-O-(CH_2)_2-O-(CH_2)_3-NH_2$ II

$H_2N-CH_2-CH_2-O-CH_2-CH_2-NH_2$ III

$H_2N-[CH_2-CH_2-O]_2-CH_2-CH_2-NH_2$ IV

$H_2N-(CH_2)_4-O-(CH_2)_4-NH_2$ V

$H_2N-(CH_2)_3-O-(CH_2)_4-O(CH_2)_3-NH_2$ VI

Diamine der Formel II haben besondere technische Bedeutung erlangt.

Die verwendeten Diamine haben erfindungsgemäß einen Gehalt an Hydrazin in Form von Hydrazinhydrat. Vorteilhaft beträgt der Gehalt an Hydrazin von 5 bis 200 ppm, insbesondere von 10 bis 150 ppm.

Geeignete Dicarbonsäuren sind beispielsweise solche wie sie üblicherweise für die Herstellung von Polyamiden verwendet werden. Bevorzugt für Dicarbonsäuren sind Alkandicarbonsäuren mit 4 bis 12, insbesondere 4 bis 8 Kohlenstoffatomen. Besondere Bedeutung haben $\alpha,\omega$-Dicarbonsäuren mit der genannten Kohlenstoffzahl erlangt wie Adipinsäure, Sebacinsäure, Azelainäure oder Dodecandsäure. Andere geeignete Dicarbonsäuren sind beispielsweise Terephthalsäure oder Isophthalsäure. Die genannten Dicarbonsäuren können auch im Gemisch eingesetzt werden. Besondere Bedeutung haben Adipinsäure und Sebacinsäure erlangt.

Die Herstellung von Salzen aus Dicarbonsäuren und Diaminen ist an sich bekannt. In der Regel werden Diamine und Dicarbonsäuren in einer wäßrigen Lösung bei erhöhter Temperatur, z.B. 50 bis 95°C, umgesetzt, so daß eine 30 bis 65 gew.%ige wäßrige Lösung des Salzes erhalten wird. Die so erhaltenen wäßrigen Lösungen können direkt für die Herstellung von Polyamiden verwendet werden. Es ist auch möglich hieraus die entsprechenden Salze durch Eindampfen oder Ausfällen zu gewinnen und diese für die Herstellung von Polyamiden einzusetzen.

Polyamide, die aus den so hergestellten Salzen erhalten worden sind, eignen sich für die Herstellung von Fäden, Fasern und Formteilen.

Die Erfindung sei an folgenden Beispielen veranschaulicht.

**Beispiel**

Adipinsäure in Form einer 50 gew.%igen wäßrigen Lösung wurde mit der äquivalenten Menge von 4,7-Dioxadecan-1,10-diamin bei einer Temperatur von 90°C umgesetzt. Die mit unterschiedlichen Mengen an Hydrazin in Form von Hydrazinhydrat erzielten Ergebnisse werden in folgender Tabelle aufgeführt.

**Tabelle**

|  | ohne Zusatz | 25 ppm Hydrazin | 50 ppm Hydrazin | 100 ppm Hydrazin |
|---|---|---|---|---|
| Eigenfarbe (1) | 7 | 5 | 4 | 2 |
| Vergilbung (2) | 156 | 60 | 23 | 16 |
| UV-Zahl (3) | 446 | 374 | 273 | 227 |
| Perjodatzahl (4) | 0,111 | 0,055 | 0,021 | 0,007 |
| UV-Zahl nach 30 min bei 90°C | 447 | 469 | 365 | 275 |

1) Eigenfarbe: APHA gemessen bei 90°C an der 40 gew.%igen wäßrigen Lösung

2) Vergilbung: APHA einer 40 gew.%igen wäßrigen Lösung nach 24stündiger Temperierung auf 85°C. Bestimmung der Extinktion mittels eines Elko II Photometers mit S 47 und J 62 Filter bei einer Schichtdicke von 5 cm. Mittels einer Eichkurve ergibt sich APHA aus Extinktion S 47 minus Extinktion J 62.

3) UV-Zahl: Summe der Extinktionen bei 226, 282 und 295 mμ × 100, gemessen an einer 40 gew.%igen wäßrigen Lösung bei 25°C gegen bidestilliertes Wasser in 10 cm Schichtdicke.

4) Perjodatzahl: 50 g einer 40 gew.%igen wäßrigen Lösung des Salzes werden mit 1 ml einer 0,5 gew.%igen wäßrigen Kaliumperjodatlösung versetzt, 30 Minuten auf 90°C erhitzt, dann auf Raumtemperatur abgekühlt und in einer Schichtdicke von 5 cm mit einem Elko II Photometer mit einem Filter S 45 die Extinktion gemessen

**Ansprüche**

1. Verwendung von Diaminen der Formel

$$H_2N-(R^1-O)_n-R^2-NH_2 \quad I$$

in der $R^1$ und $R^2$ jeweils gleich oder verschieden sein können und jeweils für einen Alkylenrest mit 2 bis 4 Kohlenstoffatomen stehen und n eine ganze Zahl von 1 bis 3 bedeutet, zur Herstellung von Salzen mit Dicarbonsäuren, dadurch gekennzeichnet, daß die Diamine der Formel I einen Gehalt an Hydrazin in Form von Hydrazinhydrat haben.

2. Verwendung von Diaminen der Formel I nach Anspruch 1, dadurch gekennzeichnet, daß sie einen Gehalt von 5 bis 200 ppm an Hydrazin in Form von Hydrazinhydrat haben.

3. Verwendung von Diamin der Formel II

$$H_2N-(CH_2)_3-O-(CH_2)_3-O-(CH_2)_3-NH_2 \quad II$$

mit einem Gehalt von 10 bis 150 ppm Hydrazin in Form von Hydrazinhydrat zur Herstellung von Salzen mit Dicarbonsäuren.